# EUROPEAN PATENT APPLICATION

(11) **EP 2 135 547 A2**
(43) Date of publication of application: **23.12.2009**
(21) Application number: 09162828.9
(22) Date of filing: 16.06.2009
(51) Int. Cl.: A61B 5/0205

(54) **Method, system and apparatus for monitoring patients**

(30) Priority: 17.06.2008 US 140403
(71) Applicant: General Electric Company, Schenectady, NY 12345 (US)
(72) Inventor: RANTALA, Borje, 00670, Helsinki (FI)
(74) Representative: Illingworth-Law, William Illingworth

(57) **Abstract**

A method, system and apparatus for monitoring a subject (10) are disclosed. The subject is provided with a plurality of monitoring units including a fixed monitoring unit (14) at a predetermined location and a mobile monitoring unit (15) attached to the subject (10), wherein the fixed monitoring unit (14) fails to have separate measurement elements attachable to the subject. First physiological data is acquired from the subject through the fixed monitoring unit (14) and second physiological data through the mobile monitoring unit (15). A least one state index indicative of the state of the subject is produced based on first selected physiological data. The producing includes employing second selected physiological data to enhance the reliability of the at least one state index when both the first physiological data and the second physiological data are available from the subject, wherein the first selected physiological data belongs to one of the first and second physiological data and the second selected physiological data belongs to one of the first and second physiological data and fails to belong to the first selected physiological data.

## Description

### BACKGROUND OF THE INVENTION

This disclosure relates to a method and system where an individual is monitored with multiple monitoring devices that provide various physiological data from the said individual. This disclosure also relates to an apparatus used in the system.

In a hospital environment, monitoring of a critically ill patient requires a plurality of different monitoring devices so that the physical parameters vital for tracking the state of the patient may be obtained continuously and in a reliable manner. The monitoring requires sophisticated monitoring devices and a rather complex measurement set-up with a plurality of sensing elements being attached to the patient who stays put in his/her hospital bed.

Although patients with minor ailments do not have to be monitored as efficiently as patients in critical care, it would be desirable that such patients could also be under constant supervision of the nursing staff. This is because various health-related incidents, which result in increased morbidity/mortality, happen even to these patients. Earlier detection of such incidents could enable the nursing staff to improve patient outcomes.

However, continuous patient monitoring/tracking is technically difficult to implement for all patients, since patients that are not critically ill are not normally tied to their beds but may move around in non-critical care areas and visit different hospital areas, such as the hospital canteen. This problem is further aggravated by the fact that obtaining reliable measures from such patients often requires a static measurement set-up and possibly also the presence of nursing staff, since reliable measures cannot normally be obtained by patient-wearable sensing devices that do not excessively interfere with the normal activities of such patients and would thus otherwise be suitable for monitoring roaming patients.

### BRIEF DESCRIPTION OF THE INVENTION

The above-mentioned problems are addressed herein which will be comprehended from the following specification.

In an embodiment, a method for monitoring a subject comprises providing the subject to be monitored with a plurality of monitoring units including a fixed monitoring unit at a predetermined location and a mobile monitoring unit attached to the subject, wherein the fixed monitoring unit fails to have separate measurement elements attachable to the subject. The method further comprises acquiring first physiological data from the subject through the fixed monitoring unit and second physiological data from the subject through the mobile monitoring unit and producing at least one state index based on first selected physiological data, wherein the at least one state index is indicative of the state of the subject and wherein the producing includes employing second selected physiological data to enhance reliability of the at least one state index, when both the first physiological data and the second physiological data are available from the subject, and wherein the first selected physiological data belongs to one of the first and second physiological data and the second selected physiological data belongs to one of the first and second physiological data and fails to belong to the first selected physiological data.

In another embodiment, a system for monitoring a subject comprises a fixed monitoring unit at a predetermined location and a mobile monitoring unit attachable to the subject, wherein the fixed monitoring unit fails to have separate measurement elements attachable to the subject and wherein the fixed monitoring unit is configured to acquire first physiological data from the subject and the mobile monitoring unit is configured to acquire second physiological data from the subject. The system further comprises an index determination unit configured to produce at least one state index based on first selected physiological data, wherein the at least one state index is indicative of the state of the subject, and wherein the index determination unit is configured to employ second selected physiological data to enhance reliability of the at least one state index when both the first physiological data and the second physiological data are available from the subject, and wherein the first selected physiological data belongs to one of the first and second physiological data and the second selected physiological data belongs to one of the first and second physiological data and fails to belong to the first selected physiological data.

In a still further embodiment, an apparatus for monitoring a subject comprises a receiver configured to receive first physiological data from a fixed monitoring unit and second physiological data from a mobile monitoring unit attachable to a subject, the first and second physiological data being obtained from the subject. The apparatus is further configured to produce at least one state index based on first selected physiological data, the at least one state index being indicative of the state of the subject; and to employ second selected physiological data to enhance reliability of the at least one state index when both the first physiological data and the second physiological data are received, wherein the first selected physiological data belongs to one of the first and second physiological data and the second selected physiological data belongs to one of the first and second physiological data and fails to belong to the first selected physiological data.

Various other features, objects, and advantages of the invention will be made apparent to those skilled in the art from the following detailed description and accompanying drawings.

### BRIEF DESCRIPTION OF THE DRAWINGS

FIG. 1 is a schematic diagram illustrating one embodiment of a patient monitoring system when the patient is in hospital bed;
FIG. 2 is a flow diagram illustrating the monitoring of a patient within a hospital;
FIG. 3 is a schematic diagram illustrating the monitoring of the patient in the embodiment of FIG. 1 when the patient is away from his/her bed;
FIG. 4 illustrates an example of the enhancement of the reliability of two patient-specific state indices; and
FIG. 5 shows an example of the spectra obtained from the fixed and mobile monitors.

### DETAILED DESCRIPTION OF THE INVENTION

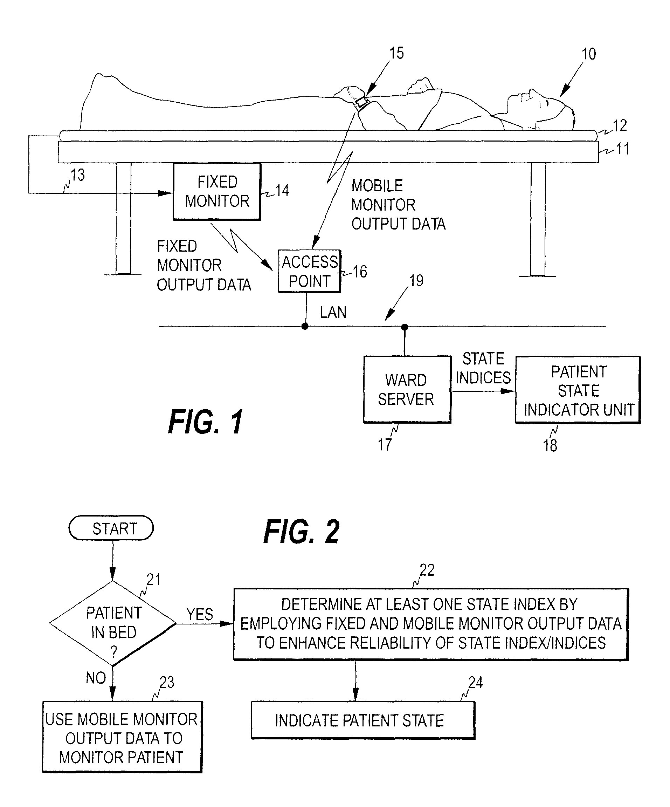

Referring to FIG. 1, a patient monitoring system comprises a fixed monitor 14 and a mobile monitor 15 attachable to a patient 10. Both the fixed and the mobile monitor measure, independently from each other, physiological data from the patient. The fixed monitor performs measurements when the patient is at the fixed monitor, while the mobile monitor may perform measurements independently of the location of the patient within a certain area, such as the premises of a hospital.

In the embodiments discussed below, each of the monitors is provided with data processing capability, i.e. the physiological signal data obtained from the patient is processed in the monitor to obtain one or more physiological parameters or variables. In addition to the physiological parameters, the monitors may also output the physiological signal data and/or other parameters, such as the identity of the bed or patient. Although the output data of the monitors may vary, it is desirable that pulse rate, oxygen saturation, and respiration rate are monitored for all patients. Therefore, the embodiments discussed below relate to the determination of these parameters.

The fixed monitor comprises in this example a bed monitor 14 provided with a recording mattress 12 in a hospital bed 11. The recording mattress 12 records the movements of the patient lying on the mattress. The signal output from the recording mattress is commonly termed a ballistogram, which is an aggregate signal indicative of the various movements of the patient, such as the movements caused by respiration and cardiac mechanic function. A ballistographic recording is beneficial in the sense that no sensing elements, such as electrodes, need to be connected to the patient. The ballistographic signal 13 is obtained as long as the patient lies in the bed.

The ballistographic signal 13 obtained from the recording mattress 12 is supplied to the bed monitor 14, which is configured to derive at least one first physiological signal or parameter from the original ballistographic signal. In this example, the bed monitor derives three different signals from the ballistographic signal: a ballistocardiogram indicative of the cardiac function of the patient, a ballistorespirogram indicative of the respiration of the patient, and a motion signal indicative of other movements of the patient. As discussed below, the bed monitor may further determine an estimate of the pulse rate from the ballistocardiogram and an estimate of the respiration rate of the patient from the ballistorespirogram. Any known method, such as filtering or spectral analysis, may be used to obtain the signals and the resulting parameters from the ballistographic signal.

In addition to the above physiological signals and parameters, the output data of the fixed monitor may contain the identity of the bed in question.

The mobile monitor comprises in this example a pulse oximeter that includes a finger probe (not shown) and a wrist unit 15 attached around the wrist of the patient. The wrist unit receives (photo)plethysmographic signals from the finger probe and determines an SpO₂ value based on the signals. The wrist unit may further output the pulse rate of the patient, and an estimate for the respiration rate of the patient. The pulse rate and the estimate of the respiration rate may be obtained from the (photo)plethysmographic signals in any known manner. Normally, the highest spectral peak of the plethysmographic signal represents the pulse rate and it may thus be selected as the pulse frequency. The respiration rate of the patient may be determined based on the extra modulation that respiration causes in the plethysmographic signal. The modulation caused by the respiration of the patient may be seen in three different features of the plethysmographic signal. First, the amplitude of the component pulsating at the heart rate varies according to the respiration. Second, the pulse-to-pulse interval, i.e. the interval between successive heart beats, varies according to respiration, the said interval being often shorter during inspiration phases than during expiration phases. Third, the DC baseline signal, i.e. the top or bottom envelope of the plethysmographic signal, varies slowly according to the respiration. However, the reliability of a respiration rate measured from plethysmographic signals is compromised, and therefore it is not as useful as an estimate of the respiration rate derived from a ballistographic signal.

In addition to the above physiological parameters, the wrist unit may output the identity and location of the patient. As discussed below, the location determination of the wrist unit may be implemented in a known manner.

FIG. 2 illustrates the co-operation of the fixed and mobile monitors of FIG. 1 for monitoring the state of the patient. It is first determined at step 21, whether output data is available from both monitors, i.e. whether the patient is currently in his/her hospital bed so that a ballistographic signal is received from the recording mattress. If this is the case, the system determines at least one state index indicative of the state of the patient. In the determination process, the system determines the at least one state index based on fixed or mobile monitor output data but employs both fixed and mobile monitor output data to enhance the reliability of the said at least one state index (step 22). The system then indicates the state of the patient by displaying the state index/indices determined (step 24). If it is detected at step 21 that the patient is out of the bed, the system uses the mobile monitor output data to monitor the state of the patient (23).

In the embodiment of FIG. 1, the enhancement step 22 is carried out in a centralized unit connected to the local area network 19 of the hospital. Each of the two monitors may transmit its output data through the local area network to a predetermined centralized unit, which may be, for example, a ward server 17 located in a control room of the ward where the patient resides. As is shown in FIG. 1, both monitors may access the local area network wirelessly through the access point 16 serving the area where the bed is located. Alternatively, the fixed monitor may be provided with a wired connection to the local area network.

Prior to step 22, the ward server compares the identities received from the monitors to ascertain that the two sets of data are from the the same patient.

Having performed step 22, the ward server may indicate the resulting patient-specific state indices to the end-user through a patient state indicator unit 18.

In another embodiment, the enhancement step 22 is carried out in the fixed monitor. In this case, step 22 may be entered for example so that when the bed monitor receives the ballistographic signal from the recording mattress, it also monitors whether it is able to receive the physiological data generated by the mobile monitor of the patient. The mobile monitor may send its output data to the fixed monitor directly or through the local area network. Thus, the two monitors may also communicate directly with each other. The monitors may also set up an ad-hoc network with each other.

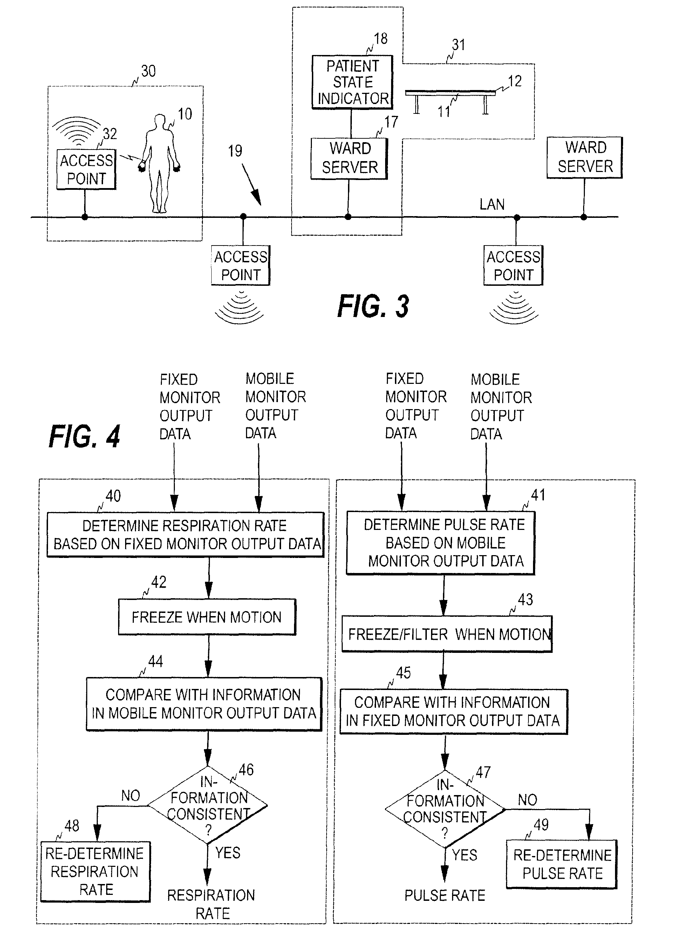

If the patient is currently away from his/her hospital bed, i.e., if the ballistographic signal is not received from the recording mattress, the system uses the output parameter(s) of the mobile monitor to monitor the state of the patient, cf. step 23 in FIG. 2. The output data of the mobile monitor is routed to the network element where the enhancing step 22 is carried out. In the example of FIG. 1, the output data of the mobile monitor is transmitted to the ward server through the access point that serves the area where the patient is currently located. FIG. 3 illustrates this situation assuming that the patient 10 is currently visiting the hospital canteen 30. The local area network of the hospital comprises a plurality of wireless access points through which the mobile monitors may communicate with the hospital devices connected to the local area network. As is known in the art, the location of the mobile device in a wireless local area network (WLAN) environment may be determined as the location of the access point with which the mobile monitor communicates. In this example, the mobile monitor 15 of the patient accesses the hospital WLAN through the access point 32 of the canteen to transmit its identity and the associated physiological data to the ward server 17 of the ward 31 where the patient resides. From the identity of the forwarding access point 32 the ward server knows that the patient is currently in the canteen. The nursing staff may thus track the location and the current mobile monitor output parameters of the patient from the ward indicator unit 18.

In a further embodiment, the output data of mobile monitor is not used directly in step 23 but is processed outside the monitor to indicate the state of the patient roaming around in the hospital. For example, the pulse rate and/or the respiration rate may be determined in the ward server based on the plethysmographic signals transmitted by the mobile monitor.

The above monitoring may be carried out for a plurality of patients at the same time. Each ward server and the associated indicator unit(s) may reside in a control room where the nursing staff may control all patients resident in the ward. In this way, the patients may be monitored substantially continuously even when they are away from their beds.

As discussed above, it is desirable that pulse rate, oxygen saturation, and respiration rate are monitored for all patients. However, it is difficult to measure the respiration of a moving patient, since the measurement normally requires various sensors and/or belts attached to the nose and/or chest of the patient, which impede the activities of the patient. The above use of a recording mattress in the fixed monitor and a pulse oximeter in the mobile monitor for implementing the recording of the said parameters rests on the discovery that most respiration-related problems occur in sleep. Therefore, it is only necessary to obtain a reliable measure of the respiration rate when the patient is in bed. The above use of the fixed and mobile monitors enables this, without a need to use wired electrodes or other sensing elements attached to the patient and without the presence of the nursing staff. Although the reliability of the respiration rate obtained from a ballistographic signal is compromised, the reliability may be enhanced by the output data of the mobile monitor, thereby to obtain a reliable measure of the respiration rate when the patient is in bed. As discussed above, an estimate of the respiration rate may also be determined when the patient is away from his/her bed. However, as the estimate is based on the plethysmographic signals, its reliability is compromised.

FIG. 4 illustrates one embodiment for enhancing the reliability of the state indices in step 22. It is assumed here that the state indices for which the enhancement step is employed are indicative the respiration and pulse rate of the patient. In this example, at least the respiration rate and a motion signal indicative of the motion of the patient are derived from the ballistographic signal. It is further assumed that at least the pulse rate is derived from the (photo)plethysmographic signals of the mobile monitor. The mobile monitor may further be provided with an acceleration transducer thereby to output a motion signal indicative of the motion of the patient.

The respiration rate of the patient is first determined based on the ballistographic signal (step 40). As discussed above, this may be carried out in the fixed monitor or in the ward server, for example. The respiration rate so obtained may be frozen when motion is detected (step 42). The respiration rate is then compared with relevant information in or derivable from the mobile monitor output data (step 44). The purpose of the comparison step is in this example to detect potential errors occurred in the determination step 40. The comparison step may involve, for example, checking that the pulse rate derived or derivable from the plethysmographic signals is consistent with the respiration rate derived from the ballistographic signal. This may be implemented for example so that the system checks if the determination of the respiration rate in step 40 has been carried out so that the signal component corresponding to the pulse frequency of the patient is erroneously selected as the respiration signal component, which is a risk especially in case of neonates for whom the said two frequencies may be close to each other. If the comparison indicates that the information obtained from the mobile monitor is consistent with the respiration rate determined, i.e., that the said error has not occurred in step 40, it is decided that the respiration rate is reliable and may thus be used as an estimate of the respiration rate of the patient. In the opposite case the respiration rate is redetermined (step 48), now avoiding the selection of the pulse rate indicated by the output data of the mobile monitor.

The pulse rate of the patient is first determined based on the plethysmographic signals of the mobile monitor (step 41). As discussed above, this may be carried out in the mobile monitor or in the ward server, for example. The pulse rate so obtained may be frozen or filtered when motion is detected (step 43). The pulse rate is then compared with relevant information in or derivable from the fixed monitor output data (step 45). The comparison step may involve, for example, checking that the respiration frequency derivable from the ballistorespirogram, and the harmonics thereof, are consistent with the pulse rate derived. This is performed to ascertain that the determination of the pulse rate in step 41 has not been carried out erroneously by selecting the respiration frequency or its harmonic component as the pulse rate. Normally the second, third, and fourth harmonic components of the respiration frequency may be potential error sources for the selection of the pulse frequency from the signal spectrum. If the pulse frequency deviates from these frequencies, consistency is found and the pulse rate determined in step 41 may be used as an estimate of the pulse rate of the patient. However, if the pulse frequency corresponds to one of these potential error sources, the pulse rate is redetermined (step 49), now avoiding the selection of the said frequencies as the pulse frequency. The problem with respiration artefacts in oximetry is known from prior art.

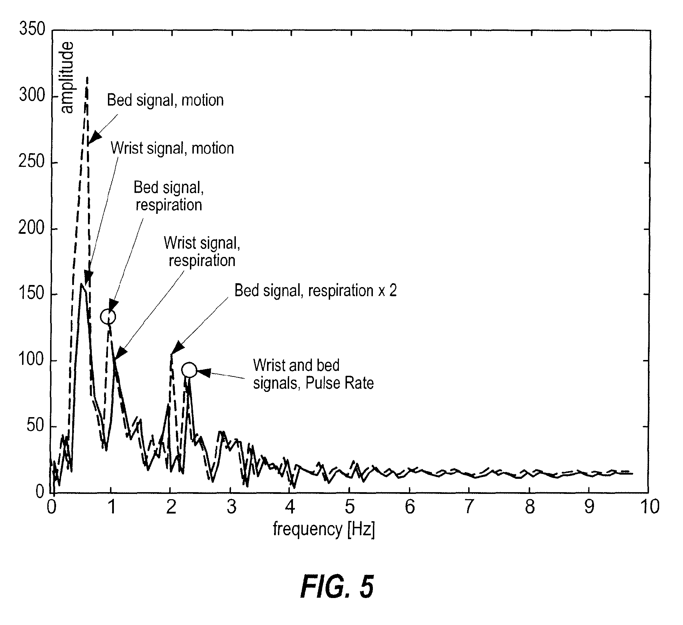

FIG. 5 illustrates an example of the spectra measured from the fixed and mobile monitors when a subject is lying on a bed. The broken line shows the spectrum of the ballistographic signal obtained from the recording mattress, while the solid line shows the spectrum of the photoplethysmographic signal obtained from a pulse oximeter attached to the wrist of the subject. The true respiration and pulse rates have been indicated by circles. In this example, the second harmonic of the respiration frequency is a potential error source for the determination of the pulse rate, since the said second harmonic is very close to the true pulse rate.

Although the above examples relate to non-critical care areas of a hospital, the monitoring may be carried in various nursing environments, such as home care and hospital incubators, where one or more subjects may be monitored. Thus, the patient may also be unable to move away from the bed. The number of the monitoring units may also be greater than two. It is also beneficial to incorporate more physiological parameters either in the mobile unit, typically ECG for heart rate, or the fixed unit, typically noninvasive blood pressure, and to use the pulse/heart rates from these in an analogous fashion to improve the performance of the fixed or mobile unit.

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural or operational elements that do not differ from the literal language of the claims, or if they have structural or operational elements with insubstantial differences from the literal language of the claims.

Aspects of the present invention are defined in the following numbered clauses:
1. A method for monitoring a subject, the method comprising:
   - providing a subject to be monitored with a plurality of monitoring units including a fixed monitoring unit at a predetermined location and a mobile monitoring unit attached to the subject, wherein the fixed monitoring unit fails to have separate measurement elements attachable to the subject;
   - acquiring first physiological data from the subject through the fixed monitoring unit and second physiological data from the subject through the mobile monitoring unit;
   - producing at least one state index based on first selected physiological data,
   wherein the at least one state index is indicative of the state of the subject and wherein the producing includes employing second selected physiological data to enhance reliability of the at least one state index, when both the first physiological data and the second physiological data are available from the subject, and wherein the first selected physiological data belongs to one of the first and second physiological data and the second selected physiological data belongs to one of the first and second physiological data and fails to belong to the first selected physiological data.
2. The method according to clause 1, wherein the employing includes eliminating potential error sources from the producing of the at least one state index.
3. The method according to clause 1, wherein
   - the acquiring includes acquiring the first physiological data and the second physiological data, wherein the first physiological data includes a first estimate of the respiration rate of the subject and the second physiological data includes a first estimate of the pulse rate of the subject;
   - the producing includes selecting the first estimate of the respiration rate as a candidate for a state index; and
   - the employing includes comparing the first estimate of the respiration rate with the first estimate of the pulse rate, thereby to check whether the first estimate of the respiration rate can be selected as the state index, wherein the state index belongs to the at least one state index.
4. The method according to clause 3, wherein the employing includes determining a new estimate of the respiration rate if the comparing indicates that the first estimate of the respiration rate corresponds to the first estimate of the pulse rate.
5. The method according to clause 1, wherein
   - the acquiring includes acquiring the first physiological data and the second physiological data, wherein the first physiological data includes respiration frequency of the subject and the second physiological data includes a first estimate of the pulse rate of the subject;
   - the producing includes selecting the first estimate of the pulse rate as a candidate for a state index; and
   - the employing includes comparing the first estimate of the pulse rate with at least one frequency of a frequency set including the respiration frequency and at least one harmonic frequency of the respiration frequency, thereby to check whether the first estimate of the pulse rate can be selected as the state index, wherein the state index belongs to the at least one state index.
6. The method according to clause 5, wherein the employing includes determining a new estimate of the pulse rate if the comparing indicates that the first estimate of the pulse rate corresponds to one of the frequencies in the frequency set.
7. The method according to clause 1, wherein the providing includes providing the subject with the fixed monitoring unit and with the mobile monitoring unit, wherein the mobile monitoring unit comprises a pulse oximeter and the fixed monitoring unit comprises a recording mattress configured to output a ballistographic signal when the subject is lying on the recording mattress.
8. The method according to clause 1, wherein the producing includes producing the at least one state index based on first selected physiological data, wherein the first selected physiological data belongs to the second physiological data when the subject is further away from the predetermined location.
9. The method according to clause 8, wherein
   - the acquiring includes acquiring second physiological data from the subject, in which the second physiological data comprises plethysmographic signal data; and
   - the producing includes producing the at least one state index, in which the at least state index includes an index indicative of arterial oxygen saturation and an index indicative of pulse rate of the subject.
10. A system for monitoring a subject, the system comprising:
   - a fixed monitoring unit at a predetermined location and a mobile monitoring unit attachable to the subject, wherein the fixed monitoring unit fails to have separate measurement elements attachable to the subject and wherein the fixed monitoring unit is configured to acquire first physiological data from the subject and the mobile monitoring unit is configured to acquire second physiological data from the subject;
   - an index determination unit configured to produce at least one state index based on first selected physiological data, wherein the at least one state index is indicative of the state of the subject, and wherein the index determination unit is configured to employ second selected physiological data to enhance reliability of the at least one state index when both the first physiological data and the second physiological data are available from the subject, and
   wherein the first selected physiological data belongs to one of the first and second physiological data and the second selected physiological data belongs to one of the first and second physiological data and fails to belong to the first selected physiological data.
11. The system according to clause 10, wherein the fixed monitoring unit comprises a recording mattress configured to output a ballistographic signal.
12. The system according to clause 11, wherein the fixed monitoring unit is configured to acquire the first physiological data, wherein the first physiological data comprises an estimate of the respiration rate of the subject.
13. The system according to clause 10, wherein the mobile monitoring unit comprises a pulse oximeter.
14. The system according to clause 13, wherein the mobile monitoring unit is configured to acquire the second physiological data, wherein the second physiological data comprises an estimate of the pulse rate of the subject.
15. The system according to clause 10, wherein the index determination unit is operatively connected to a local area network.
16. The system according to clause 15, wherein the index determination unit comprises a ward server connected to an indication unit configured to indicate the at least one state index to an end-user.
17. The system according to clause 10, wherein the fixed monitoring unit comprises the index determination unit.
18. The system according to clause 17, further comprising an indication unit connected to the fixed monitoring unit through a local area network, wherein the indication unit is configured to indicate the at least one state index to an end-user.
19. An apparatus for monitoring a subject, the apparatus comprising a receiver configured to receive first physiological data from a fixed monitoring unit and second physiological data from a mobile monitoring unit attachable to a subject, the first and second physiological data being obtained from the subject,
   wherein the apparatus is configured to
   - produce at least one state index based on first selected physiological data, the at least one state index being indicative of the state of the subject; and
   - employ second selected physiological data to enhance reliability of the at least one state index when both the first physiological data and the second physiological data are received, wherein the first selected physiological data belongs to one of the first and second physiological data and the second selected physiological data belongs to one of the first and second physiological data and fails to belong to the first selected physiological data.
20. The apparatus according to clause 19, wherein the apparatus is connected to a local area network for receiving the first and second physiological data from the local area network.

## Claims

1. A method for monitoring a subject, the method comprising:
- providing a subject (10) to be monitored with a plurality of monitoring units including a fixed monitoring unit (14) at a predetermined location and a mobile monitoring unit (15) attached to the subject (10), wherein the fixed monitoring unit fails to have separate measurement elements attachable to the subject;
- acquiring first physiological data from the subject through the fixed monitoring unit (14) and second physiological data from the subject through the mobile monitoring unit (15);
- producing at least one state index based on first selected physiological data, wherein the at least one state index is indicative of the state of the subject (10) and wherein the producing includes employing second selected physiological data to enhance reliability of the at least one state index, when both the first physiological data and the second physiological data are available from the subject, and wherein the first selected physiological data belongs to one of the first and second physiological data and the second selected physiological data belongs to one of the first and second physiological data and fails to belong to the first selected physiological data.

2. The method according to claim 1, wherein the employing includes eliminating potential error sources from the producing of the at least one state index.

3. The method according to claim 1 or claim 2, wherein
- the acquiring includes acquiring the first physiological data and the second physiological data, wherein the first physiological data includes a first estimate of the respiration rate of the subject (10) and the second physiological data includes a first estimate of the pulse rate of the subject;
- the producing includes selecting the first estimate of the respiration rate as a candidate for a state index; and
- the employing includes comparing (44) the first estimate of the respiration rate with the first estimate of the pulse rate, thereby to check whether the first estimate of the respiration rate can be selected as the state index, wherein the state index belongs to the at least one state index.

4. The method according to any one of the preceding claims, wherein the employing includes determining a new estimate of the respiration rate if the comparing indicates that the first estimate of the respiration rate corresponds to the first estimate of the pulse rate.

5. The method according to any one of the preceding claims, wherein
- the acquiring includes acquiring the first physiological data and the second physiological data, wherein the first physiological data includes respiration frequency of the subject and the second physiological data includes a first estimate of the pulse rate of the subject (10);
- the producing includes selecting the first estimate of the pulse rate as a candidate for a state index; and
- the employing includes comparing (45) the first estimate of the pulse rate with at least one frequency of a frequency set including the respiration frequency and at least one harmonic frequency of the respiration frequency, thereby to check whether the first estimate of the pulse rate can be selected as the state index, wherein the state index belongs to the at least one state index.

6. The method according to any one of the preceding claims, wherein the employing includes determining a new estimate of the pulse rate if the comparing indicates that the first estimate of the pulse rate corresponds to one of the frequencies in the frequency set.

7. The method according to any one of the preceding claims, wherein the producing includes producing the at least one state index based on first selected physiological data,
wherein the first selected physiological data belongs to the second physiological data when the subject (10) is further away from the predetermined location.

8. A system for monitoring a subject (10), the system comprising:
- a fixed monitoring unit (14) at a predetermined location and a mobile monitoring unit (15) attachable to the subject, wherein the fixed monitoring unit fails to have separate measurement elements attachable to the subject and wherein the fixed monitoring unit is configured to acquire first physiological data from the subject and the mobile monitoring unit is configured to acquire second physiological data from the subject;
- an index determination unit (14, 17) configured to produce at least one state index based on first selected physiological data, wherein the at least one state index is indicative of the state of the subject (10), and wherein the index determination unit is configured to employ second selected physiological data to enhance reliability of the at least one state index when both the first physiological data and the second physiological data are available from the subject, and
wherein the first selected physiological data belongs to one of the first and second physiological data and the second selected physiological data belongs to one of the first and second physiological data and fails to belong to the first selected physiological data.

9. The system according to claim 8, wherein the fixed monitoring unit (14) comprises a recording mattress (12) configured to output a ballistographic signal.

10. The system according to claim 8 or claim 9, wherein the fixed monitoring unit (14) is configured to acquire the first physiological data, wherein the first physiological data comprises an estimate of the respiration rate of the subject (10).

11. The system according to any one of claims 8 to 10, wherein the mobile monitoring unit (15) comprises a pulse oximeter.

12. The system according to any one of claims 8 to 11, wherein the mobile monitoring unit (15) is configured to acquire the second physiological data, wherein the second physiological data comprises an estimate of the pulse rate of the subject (10).

13. The system according to any one of claims 8 to 12, wherein the index determination unit (14, 17) is operatively connected to a local area network (19).

14. The system according to any one of claims 8 to 13, wherein the fixed monitoring unit (14) comprises the index determination unit.

15. An apparatus for monitoring a subject (10), the apparatus comprising a receiver configured to receive first physiological data from a fixed monitoring unit (14) and second physiological data from a mobile monitoring unit (15) attachable to the subject (10), the first and second physiological data being obtained from the subject,
wherein the apparatus is configured to
- produce at least one state index based on first selected physiological data, the at least one state index being indicative of the state of the subject; and
- employ second selected physiological data to enhance reliability of the at least one state index when both the first physiological data and the second physiological data are received,
wherein the first selected physiological data belongs to one of the first and second physiological data and the second selected physiological data belongs to one of the first and second physiological data and fails to belong to the first selected physiological data.
